# EUROPEAN PATENT APPLICATION

(11) **EP 2 992 894 A1**
(43) Date of publication of application: **09.03.2016**
(21) Application number: 15183168.2
(22) Date of filing: 31.08.2015
(51) Int. Cl.: A61K 38/40, A61K 35/74, A61P 31/04, A61P 31/10, A61P 31/12

(54) **VAGINAL FORMULATIONS FOR PREVENTING AND TREATING VAGINAL AND CERVICO-VAGINAL INFECTIONS**

(30) Priority: 05.09.2014 IT MI20141542
(71) Applicant: Progine Farmaceutici S.r.l., 50040 Calenzano (FI) (IT)
(72) Inventor: LANDI, Lapo, 50013 Campi Bisenzio FI (IT)
(74) Representative: Asensio, Raffaella Consuelo

(57) **Abstract**

Association of lactoferrin and at least one probiotic for the treatment of vaginal and cervical-vaginal infections of the bacterial, viral, and mycotic type and in particular for the treatment of the aforementioned infections that can induce preterm birth and/or premature breaking of the membranes, as well as relative formulations suitable for vaginal administration, which contain the aforementioned association in combination with suitable excipients and/or diluents.

## Description

### FIELD OF THE INVENTION

The present invention concerns an association of lactoferrin and at least one probiotic for use in the treatment and prevention of vaginal and cervical-vaginal infections of the bacterial, viral and mycotic type, which in gestation can induce preterm birth and/or premature breaking of the membranes. The present invention concerns vaginal formulations containing the aforementioned association.

### STATE OF THE ART

Lactoferrin is a polypeptide belonging to the family of transferrins and with a molecular weight of about 80 kDa. Lactoferrin is capable of coordinating the ferric ion (Fe³⁺) through two binding sites and normally has a ferric content that is variable but always less than the maximum possible saturation.

In nature, lactoferrin is found particularly in milk, but it is present in many mucous secretions like tears and saliva. The antimicrobial activity of lactoferrin is linked to its affinity for Fe³⁺. The combination of lactoferrin with the ferric ion in mucous secretions modulates the activity and the aggregation ability of bacteria and of viruses towards the cellular membranes, since lactoferrin removes the iron necessary for the cellular replication of some bacteria from the environment. Some bacteria require iron to carry out cellular replication and lactoferrin, on the other hand, removes it from the surrounding environment, preventing the proliferation thereof.

Lactoferrin is normally contained in neutrophil granulocytes. In humans, the gene encoding lactoferrin is located on chromosome 3 with localisation 3q21-q23. Lactoferrin also possesses a bactericide activity independent from its ability to bind iron ions, since it is able to attack and lyse he bacterial membrane, exploiting the affinity of its cationic domains towards the bacterial membrane (negatively charged). This activity, in combination with lysozyme, an enzyme capable of breaking the β1-4 glycosidic bonds of peptidoglycan, leads to the death of the bacterium by cytolysis. The use of lactoferrin as an antibacterial has been reported (WO9806425), for the treatment of anaemia (WO2004060392), within formulations for infancy (WO2012091945 and WO2010130643), as zootechnical supplement (WO03047363). It is also known to use lactoferrin in the gynecological field. For example in IT1392620, in the Applicant's name, it is disclosed to use lactoferrin to prevent infections that can induce preterm birth. In the subsequent patent application, again in the Applicant's name MI2012A0002151, it is in particular disclosed to use lactoferrin for the prevention of miscarriage following amnio- and villo-centesis.

A recent study demonstrated that the use of vaginal LF is able to increase the presence of lactobacillus at the level of the vaginal flora demonstrating that the action of the LF is also prebiotic and therefore able to promote the growth of some lactobacilli present in the vagina. (Otsuki K. et al. Administration of oral and vaginal prebiotic lactoferrin for women with a refractory vaginitis recurring preterm delivery: appearance of lactobacillus in vaginal flora followed by term delivery. J. Obstet. Gynecol. Res. 2013).

This type of activity is only found if lactoferrin is administered daily, topically, for a long time period, lasting at least 20 days.

Many studies associate the therapeutic result with the how long it is administered for. In "Journal of Obstetrics and Gynaecology, February 2013; 33: 144-148 Informa UK, it is reported that lactoferrin administered in pregnant women with shortened uterine cervix with respect to the average for the gestational period after administration with lactoferrin by vaginal route show a lengthening of the uterine cervix and a reduction of interleukin not before 30 days from the treatment start. This means that patients treated with lactoferrin are able to develop probiotics in particular lactobacilli in the vagina only after 30 days from the treatment start. These lactobacilli gradually increase until they become predominant in the vaginal flora, promoting the normal development of the pregnancy of the patient in question. However, in the first 30 days from the start of the treatment, the pregnant woman is not protected, thus being vulnerable to possible infections capable of triggering the mechanisms of preterm birth. The same applies in the case of administration of only lactobacilli, even if in recent years there has been increasing interest in the use of lactobacilli, as a substance suitable for correcting deficient conditions of the probiotic flora, particularly from when it has been demonstrated that the latter can be the cause of vaginal infections that represent a risk factor for obstetric complications.

Indeed, bacterial vaginosis hits between 9-23% of pregnant women. Numerous epidemiological studies have documented an association between vaginosis and/or vaginitis and miscarriage, preterm birth, chorioamnionitis, premature breaking of the membranes and post-birth endometritis (19).

In order to be effective, just the lactobacilli need daily administration for a few months of treatment, in any case not less than 30 days.

This is also due to the fact that lactobacilli would not be able to survive in the vaginal environment altered by infections and possible inflammations associated with them, would not be able to restore the vaginal flora.

Therefore, there is a need to have formulations for vaginal use that do not have the aforementioned drawbacks and that can therefore provide protection from possible infections much before the formulation currently on the market.

### SUMMARY OF THE INVENTION

The Applicant has now found that it is possible to avoid the aforementioned drawback to prevent and treat vaginal and cervical-vaginal infections, an association of lactoferrin and probiotic active ingredient able to be administered topically/vaginally. In particular, a further object of the present invention are formulations suitable for being administered vaginally containing the aforementioned association in combination with suitable excipients and/or diluents.

Indeed, by administering this type of association in the vagina, the probiotic(s) is/are supported by lactoferrin, which, by acting both as prebiotic and as specific support for this type of active ingredient, is able to provide a suitable environment in the vagina for its/their survival, thus providing a system of protection from infections in short time periods from the relative administration and in any case much earlier than lactoferrin allows endogenous probiotics to be produced.

The administration of the association of lactoferrin and probiotics also allows the possibility of directly administering probiotics such as the lactobacilli that, if administered as a single active ingredient in the vaginal environment, would not be able to survive in the vaginal environment altered by the presence of infections/inflammations and therefore they would not be able to restore the vaginal flora.

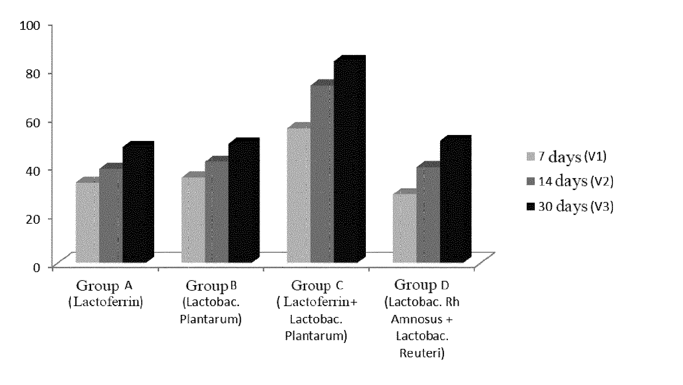

### DESCRIPTION OF FIGURE 1

Figure 1 shows in graphical form the % improvement of the symptoms of bacterial vaginosis/vaginitis in the following 4 groups of patients treated with:
   A) only lactoferrin in the form of vaginal tablets at the dose of 300 mg , 1 in the evening x 30 days (Difesan, Progine Farmaceutici Srl).
   B) Lactobacillus plantarum p17630, 1 vaginal tablet in the evening x 30 days (Gyno-Canesflor, Bayer)
   C) association of lactoferrin 300 mg and Lactobacillus plantarum LB 931, 1 vaginal tablet in the evening x 30 days (Difesan Flor, Progine Farmaceutici Srl)
   D) association of Lactobacillus rhamnosus GR-1 and Lactobacillus reuteri RC-14, 1 vaginal tablet in the evening x 30 days,
after 7 (v1), 14 (v2) and 30 (v3) days of administration.

The improvement of the symptomology was significantly (p< 0.01) greater in women treated with the association Lactoferrin and Lactobacillus Plantarum with respect to the other 3 groups.

### DETAILED DESCRIPTION OF THE INVENTION

For the purposes of the present invention, by vaginal and cervical-vaginal infections we mean vaginitis, i.e. chronic or acute inflammations of the vagina, infections of a bacterial origin such as vaginosis, of a viral origin, such as Herpes simplex and Papilloma Virus (HPV), or of a mycotic origin, such as thrush.

For example, this includes the use according to the present invention of the association lactoferrin and at least one probiotic active ingredient for the prevention of qualitative-quantitative type alterations of the physiological vaginal flora, alterations capable of causing infections and consequently inflammatory processes, which, if they occur in pregnant women, can constitute risk factors of preterm birth and/or of premature breaking of the membranes.

Therefore, a particular use of the association object of the present invention is directed to the prophylaxis of preterm birth and/or the premature breaking of the membranes. For the purposes of the present invention the term lactoferrin means:
- apolactoferrin, i.e. lactoferrin with degree of saturation ≤2%,
- lactoferrin with degree of saturation comprised between 2 and 98%,
- ololactoferrin with degree of saturation≥98%.

For the purposes of the present invention the term prebiotic means a substance that is not absorbed by the body but is characterised in that it promotes the growth and the activity of probiotic active ingredient(s).

For the purposes of the present invention probiotic active ingredient(s) means a living organism that, administered in suitable quantities, brings a benefit to the health of the host".

Probiotics, which can be contained in the association object of the present invention are in particular selected among lactobacilli, bifidobacteria and relative mixtures. Preferably as lactobacilli at least one of the following is used: *Lactobacillus plantarum, Lactobacillus bulgaricus, Lactobacillus casei, Lactobacillus bulgaricus, Lactobacillus paracasei, Lactobacillus rhamnosus, Lactobacillus acidophilus, Lactobacillus fermentum, Lactobacillus gasseri, Lactobacillus crispatus* and as bifidobacteria at least one of the following is used : *Bifidobacterium lactis, Bifidobacterium bifidum and* relative mixtures.

A particularly preferred association for use according to the present invention comprises a probiotic *Lactobacillus plantarum* LB931.

The lactoferrin used in the association object of the present invention is preferably bovine lactoferrin.

The formulations for vaginal administration, further object of the present invention, preferably contain lactoferrin in a quantity comprised between 250 and 800 mg, more preferably 300mg.

The probiotic(s) is/are preferably contained in amounts comprised between 100 million and 10 billion ufc. When *Lactobacillus plantarum* LB931 is used, it is present in a quantity equal to 1 billion ufc.

The vaginal formulations, object of the present invention, are preferably in the form of capsules, tablets, ovules and contain at least one excipient suitable for promoting their dissolution in the vagina, selected from lubricants, disaggregants, diluents, absorbents, lubricants, anti-adhesives, glidants, binders, surfactants, dyes, anti-oxidants or relative mixtures.

Even more preferably, in the composition according to the present invention the at least one excipient suitable for intravaginal delivery is selected from: mannitol, magnesium stearate, vegetable magnesium stearate, hydroxy ethyl cellulose, crospovidone, polycarbophil, sodium carboxymethyl cellulose, corn starch, silicon dioxide and mixtures thereof.

According to a particularly preferred embodiment, the vaginal formulations according to the present invention are in the form of capsules to safeguard the vitality of the at least one probiotic present in the formulation.

The composition inside the aforementioned vaginal capsules comprising the association of at least one probiotic and lactoferrin, preferably contains at least one of the aforementioned excipients: magnesium stearate, silicon dioxide and corn starch. Hereafter the clinical tests are shown wherein the effectiveness of the association object of the present invention is highlighted.

### Material, Method and Results

In order to evaluate the effectiveness of the association object of the invention in women affected by bacterial vaginosis/aerobic vaginitis, a perspective observational study was carried out on 80 women of age comprised between 20 and 35 years suffering from bacterial vaginosis and split into four groups of 20 patients each and treated as follows:
Group A: 20 women only treated with lactoferrin in vaginal tablets at the dose of 300 mg , 1 tablet every evening x 30 days (Difesan, Progine Farmaceutici Srl).
Group B : 20 women only treated with Lattobacillus plantarum p17630, 1 vaginal tablet every evening x 30 days (Gyno-Canesflor, Bayer)
Group C : 20 women treated with the association lactoferrin 300 mg and lactobacillus plantarum LB 931, 1 vaginal tablet every evening x 30 days (Difesan Flor, Progine Farmaceutici Srl).
Group D : 20 women treated with a preparation in vaginal capsules based on Lactobacillus rhamnosus GR-1 and Lactobacillus reuteri RC-14, 1 vaginal capsule every evening x 30 days ( Dicoflor Elle cps vaginali AGPharma, Roma).

Those considered eligible for the study were women with from moderate to severe vaginal symptoms (burning, itching, vaginal dryness) that were from moderate moderate to severe (on a scale of 4) : - age >20 years; -diagnosis of bacterial vaginosis (positive to 3 out of 4 Amsel's criteria or value of the Nugent score equal to 7-10); aerobic bacterial vaginitis (defined on a cultural basis and clinically (clinical symptomology, high pH (<6), high presence of leucocytes in the strip, absence thereof after the addition of potassium hydroxide to the vaginal exudate collected on the slide of aminic smell) or microscopically in accordance with Donders et al (2002), - that allowed participation in the research.

The women admitted to the study were assigned to a specific group and treated with the therapy indicated above.

An evaluation was made at the time of recruitment (V0), after 7(V1), after 14(V2) and after 30 days of therapy (V3), of the presence of vaginal itching, leukorrhea, vaginal burning and vaginal dryness using a scale including 4 levels: no, slight, moderate, severe.

At such a time the clinical pattern was defined by the absence of clue cells and of negative results for at least 2 Amsel's criteria (for bacterial vaginosis) and/or absence of clinical symptomology and normal vaginal pH or Donders score <3 (for aerobic vaginitis) and/or negative culture examination (if carried out).

The overall 80 subjects were admitted to the study. Of these 20 were treated with only Lactoferrin (Difesan); 20 with only L. Plantarum (Gyno-canesflor) and 20 with the association Lactoferrin and L. Plantarum (Difesan Flor) and 20 women with Lactobacillus rhamnosus GR-1 and Lactobacillus reuteri RC-14 (Dicoflor Elle cps vaginali AGPharma Roma).

All 80 patients were suffering from bacterial vaginosis/vaginitis. The age of the women of the various groups were able to be matched and therefore the groups were homogeneous both for the symptomology referred and for age.

The differences with regard to the base symptomological history between the four groups were not significant whereas at the evaluation after 14 days from the start of the treatment, between the four groups the frequency of moderate or severe symptomatology and the degree of vaginal dryness were significantly better in group C, i.e. in women treated with the association Lactoferrin-L. Plantarum (p<0,05).

At the visit after 7, 14 and 30 days the results, the clinical history expressed as % improvement was: 55.6%, 73.2% and 83.3% of the subjects in the group Lactoferrin-L plantarum with respect to 33.1%, 38.7 and 47.8 in the group treated with only Lactoferrin (p<0.05) ; vs 35.2%, 41.7 and 49.2% in the group treated with only L. Plantarum ; vs 28,3%, 39,4 and 50.3% in the group of women treated with Lactobacillus rhamnosus GR-1 and Lactobacillus reuteri RC-14 .

The present study demonstrated the ability of the association between Lactoferrin and Lactobacillus plantarum in improving the symptomatology of women suffering from bacterial vaginosis/vaginitis in a shorter time (halved) and in a significantly greater manner with respect to the other 3 groups evaluated (Fig 1) and in ensuring a faster and more effective resolution of symptoms linked to bacterial vaginosis and not with respect to treatment with:
- only Lactoferrin,
- with only Lactobacillus Plantarum and
- with association of Lactobacillus rhamnosus GR-1 and Lactobacillus reuteri RC-14.

The interpretation of this important scientific data demonstrates a synergic action between the two substances used, on the one hand Lactoferrin which, as well as carrying out an anti-bacterial and anti-inflammatory action, behaves as a prebiotic more rapidly promoting a better colonisation and development of the *Lactobacillus plantarum,* which, through a lowering of the vaginal pH, better counteracts the presence of the bacterial infection or other type of infection that the women suffers from.

Even more so this association results particularly useful during pregnancy, both as preventive and as therapeutic action, to reduce the risks of preterm births. In this particular situation, indeed, the woman cannot take drugs except when strictly necessary.

On the other hand, Lactoferrin and lactobacilli do not have contraindications in pregnancy or side effects that limit their use.

Therefore, treatment with Difesan Flor is considered appropriate and useful in promoting the resolution of the clinical conditions of women suffering from bacterial or anaerobic vaginitis or more generally all vaginal infections and also for the prevention of preterm births reducing the therapeutic response times with respect to applications of only lactoferrin or of only lactobacilli vaginally.

## Claims

1. Association of lactoferrin and at least one probiotic active ingredient for vaginal topical use.

2. Association for use according to claim 1, for the prevention and treatment of vaginal and cervical-vaginal infections.

3. Association for use according to claim 1 or 2, wherein said infections are of bacterial, mycotic or viral origin.

4. Association for use according to claim 3, wherein said infections are selected among vaginitis, vaginosis, herpes simplex, papilloma virus (HPV), thrush.

5. Association for use according to claim 1, for the prevention of qualitative-quantitative type alterations of the physiological vaginal flora, capable of causing infections and inflammatory processes.

6. Association for use according to claim 5 in the prophylaxis of preterm birth and/or for the prevention of breaking of the membranes.

7. Association for use according to any one of claims 1-6, wherein said at least one probiotic active ingredient is a lactobacillus or a bifidobacterium.

8. Association for use according to claim 7 wherein said at least one probiotic active ingredient is *Lactobacillus plantarum* LB931.

9. Formulation suitable for vaginal administration containing an association of lactoferrin and at least one probiotic active ingredient in combination with suitable excipients and/or diluents.

10. Formulation according to claim 8 containing between 250 and 800 mg of lactoferrin and between 100 million and 10 billion ufc of at least one probiotic active ingredient.

11. Formulation according to claims 9 or 10, containing 300 mg of lactoferrin and, as probiotic active ingredient, *Lactobacillus plantarum* LB931 in an amount equal to 1 billion ufc.

12. Formulation according to any one of claims 8-11, in the form of vaginal capsules.

13. Vaginal formulation according to claim 12 wherein the composition within the aforementioned vaginal capsules comprising the association of at least one probiotic and lactoferrin, contains at least one of the aforementioned excipients: magnesium stearate, silicon dioxide and corn starch.
